# EUROPEAN PATENT SPECIFICATION

(11) **EP 0 822 788 B1**
(45) Date of publication and mention of the grant of the patent: **04.09.2002**
(21) Application number: 96905974.0
(22) Date of filing: 01.04.1996
(51) Int. Cl.: A61F 2/06, A61L 31/00

(54) **DRUG RELEASE COATED STENT**
BESCHICHTETER DILATATOR ZUR ABGABE EINES ARZNEISTOFFS
EXTENSEUR AVEC UN REVETEMENT CAPABLE DE LIBERER UN MEDICAMENT

(30) Priority: 19.04.1995 US 424884
(43) Date of publication of application: 11.02.1998
(73) Proprietor: SCHNEIDER (USA) INC., Plymouth, Minnesota 55442 (US)
(72) Inventor: DING, Ni, Plymouth, MN 55446 (US); HELMUS, Michael, N., Long Beach, CA 90807 (US)
(74) Representative: Williams, Ceili
(86) International application number: IB9600272
(87) International publication number: WO96032907

(56) References cited:
- EP-A- 0 623 354
- WO-A-91/12779
- US-A- 3 932 627

## Description

### BACKGROUND OF THE INVENTION

### I. Field of the Invention

The present invention relates generally to elastic, self-expanding stent prostheses for lumen, e.g., vascular, implantation and, more particularly, to the provision of biostable elastomeric coatings on such stents which incorporate elutable or diffusible biologically active species for controlled release directly in the coating structure.

### II. Related Art

In surgical or other related invasive medicinal procedures, the insertion and expansion of stent devices in blood vessels, urinary tracts or other difficult to access places for the purpose of preventing restenosis, providing vessel or lumen wall support or reinforcement and for other therapeutic or restorative functions have become a common form of long-term treatment. Typically, such prostheses are applied to a location of interest utilizing a vascular catheter, or similar transluminal device, to carry the stent to the location of interest where it is thereafter released and expanded in situ. These devices are designed primarily as permanent implants which may become incorporated in the vascular or other tissue which they contact at implantation.

Stent devices of the self-expanding tubular type for transluminal implantation, then, are generally known. One type of such device includes a flexible tubular body which is composed of several individual flexible thread elements each of which extends in a helix configuration with the centerline of the body serving as a common axis. A plurality of elements having the same direction of winding but which are displaced axially relative to each other are provided which meet under crossing a like number of elements also so axially displaced but having the opposite direction of winding. This configuration provides a sort of braided tubular structure which assumes a stable dedicated diameter upon the relaxation but which can be reduced as for insertion by the application of axial tension which, in turn, produces elongation of the body with a corresponding diameter contraction that allows the stent to be conducted through the vascular system as a narrow elongated device and thereafter allowed to expand upon relaxation at the location of interest. Prostheses of the class including a braided flexible tubular body are illustrated and described in U.S. Patents 4,655,771 and 4,954,126 to Wallsten and 5,061,275 to Wallsten et al.

The general idea of utilizing implanted stents to carry medicinal agents, such as thrombolytic agents, also have been devised. U.S. Patent 5,163,952 to Froix discloses a thermal memoried expanding plastic stent device which can be formulated to carry a medicinal agent by utilizing the material of the stent itself as an inert polymeric drug carrier. Pinchuk, in U.S. Patent 5,092,877, discloses a stent of a polymeric material which may be employed with a coating associated with the delivery of drugs. Other patents which are directed to devices of the class utilizing bio-degradable or bio-sorbable polymers include Tang et al, U.S. Patent 4,916,193, and MacGregor, U.S. Patent 4,994,071. A patent to Sahatjian, Patent No. 5,304,121, discloses a coating applied to a stent consisting of a hydrogel polymer and a preselected drug in which possible drugs include cell growth inhibitors and heparin. A further method of making a coated intravascular stent carrying a therapeutic material in which a polymer coating is dissolved in a solvent and the therapeutic material dispersed in the solvent and the solvent thereafter evaporated is described in European patent application 0 623 354 A1 published 09 November 1994.

An article by Michael N. Helmus (a co-inventor of the present invention) entitled "Medical Device Design--A Systems Approach: Central Venous Catheters", 22nd International Society for the Advancement of Material and Process Engineering Technical Conference (1990) discloses surfactant-heparin complexes to be used as controlled release heparin coatings. Those polymer/drug/membrane systems require two distinct layers of function.

While many attempts have been made to incorporate drug delivery in conjunction with long-term catheter or implanted stent placement, for example, the associated release time has been generally, relatively short, measured in hours and days, and success has been limited. There remains a need for a comprehensive approach that provides long-term drug release, i.e., over a period of weeks or months, incorporated in a controlled-release system. In addition, there is a further need with respect to incorporating a drug release coating on a metallic stent. Polymeric stents, although effective, cannot equal the mechanical properties of metal stents of a like thickness. For example, in keeping a vessel open, a metallic stent is superior because stents braided of relatively fine metal can provide a good deal of strength to resist circumferential pressure. In order for a polymer material to provide the same strength characteristics, a much thicker-walled structure or heavier, denser filament weave is required. This, in turn, reduces the area available for flow through the stent and/or reduces the amount of porosity available in the stent. In addition, when applicable, it is more difficult to load such a stent onto catheter delivery systems for conveyance through the vascular system of the patient to the site of interest.

Accordingly it is a primary object of the present invention to provide a coating in a deployed stent prosthesis capable of long-term delivery of biologically active materials.

Another object of the invention is to provide a coating on a deployed stent prosthesis of optimal mechanical properties with minimal surface area for long-term delivery of biologically active therapeutic materials.

Still another object of the present invention is to provide a coating on a deployed stent prosthesis using a biostable hydrophobic elastomer in which the biologically active species is incorporated within the coating.

A still further object of the invention is to provide a deployed stent prosthesis of a siloxane polymer containing crystals of heparin for dissolution via interconnected particle interstices.

A yet still further object of the present invention is to provide a braided metallic deployed stent prosthesis having a coating of a siloxane polymer material containing an amount of dissolved and/or finely divided dexamethasone.

Other objects and advantages of the present invention will become apparent to those skilled in the art upon familiarization with the specification and appended claims.

### SUMMARY OF THE INVENTION

Many of the limitations of prior art implanted prolonged drug delivery systems associated with deployed stent prostheses are overcome by the provision of a stent for implantation in a body comprising a tubular metal body having open ends and a sidewall and a layer on a surface of this sidewall, said layer comprising a matrix material incorporating an amount of biologically active material therein for timed delivery therefrom characterised in that the sidewall is an open lattice structure with openings therein and the matrix material comprises a hydrophobic elastomeric material which permits the layer to adheringly conform to the lattice sidewall structure and so leave the openings substantially free of layer material and whereby said layer is from about 75µm to about 200µm in thickness.

Although other materials can be used including polymer materials, in the preferred embodiment, the tubular body is formed of an open braid of filaments of fine metallic wire which is axially deformable for insertion but which resumes a predetermined diameter and length upon relaxation. The metal used to form these filaments is preferably selected from the group consisting of stainless steel, titanium alloys including nitinol (a nickel titanium, thermomemoried alloy material), tantalum and cobalt-chrome alloys including cobalt-chrome-nickel alloys such as Elgiloy® and Phynox®. Further details concerning the fabrication and details of other aspects of the stents themselves may be gleaned from the above referenced U.S. Patents 4,655,771 and 4,954,126 to Wallsten and 5,061,275 to Wallsten et al. To the extent additional information contained in the above referenced patents is necessary for an understanding of the present invention, they are deemed incorporated by reference herein.

The coating layer is preferably applied as a solvent mixture of uncured polymeric material and finely divided biologically active species and then cured at an elevated temperature. The biologically active species may be at least partially soluble in the solvent mixture. The coating may be applied by dipping or spraying using evaporative solvent materials of relatively high vapour pressure to produce the desired viscosity and coating thickness.

The elastomeric material that forms the major constituent of the stent coating should possess certain properties. It is preferably a suitable hydrophobic biostable elastomeric material which does not degrade and which minimizes tissue rejection and tissue inflammation and one which will undergo encapsulation by tissue adjacent the stent implantation site. Polymers suitable for such coatings include silicones (e.g., polysiloxanes and substituted polysiloxanes), polyurethanes, thermoplastic elastomers in general, ethylene vinyl acetate copolymers, polyolefin elastomers, and EPM rubbers. The above-referenced materials are considered hydrophobic with respect to the contemplated environment of the invention.

Agents suitable for incorporation include antithrobotics, anticoagulants, antiplatelet agents, thrombolytics, antiproliferatives, antinflammatories, agents that inhibit hyperplasia and in particular restenosis, smooth muscle cell inhibitors, growth factors, growth factor inhibitors, cell adhesion inhibitors, cell adhesion promoters and drugs that may enhance the formation of healthy neointimal tissue, including endothelial cell regeneration. The positive action may come from inhibiting particular cells (e.g., smooth muscle cells) or tissue formation (e.g., fibromuscular tissue) while encouraging different cell migration (e.g., endothelium) and tissue formation (neointimal tissue).

Various combinations of polymer coating materials can be coordinated with biologically active species of interest to produce desired effects when coated on stents to be implanted in accordance with the invention. Loadings of therapeutic materials may vary. The mechanism of incorporation of the biologically active species into the surface coating, and egress mechanism depend both on the nature of the surface coating polymer and the material to be incorporated. The mechanism of release also depends on the mode of incorporation. The material may elute via interparticle paths or be administered via transport or diffusion through the encapsulating material itself.

The desired release rate profile can be tailored by varying the coating thickness, the radial distribution of bioactive materials, the mixing method, the amount of bioactive material, and the crosslink density of the polymeric material. The crosslink density is related to the amount of crosslinking which takes place and also the relative tightness of the matrix created by the particular crosslinking agent used. This, after the curing process, determines the amount of crosslinking and so the crosslink density of the polymer material. For bioactive materials released from the crosslinked matrix, such as heparin, a denser crosslink structure will result in a longer release time and small burst effect.

### BRIEF DESCRIPTION OF THE DRAWINGS

In the drawings, wherein like numerals designate like parts throughout the same:
FIGURES 1 and 1A depict greatly enlarged views of a fragment of a medical stent for use with the coating of the invention;
FIGURES 2A and 2B depict a view of a stent section as pictured in Figures 1 and 1A as stretched or elongated for insertion;

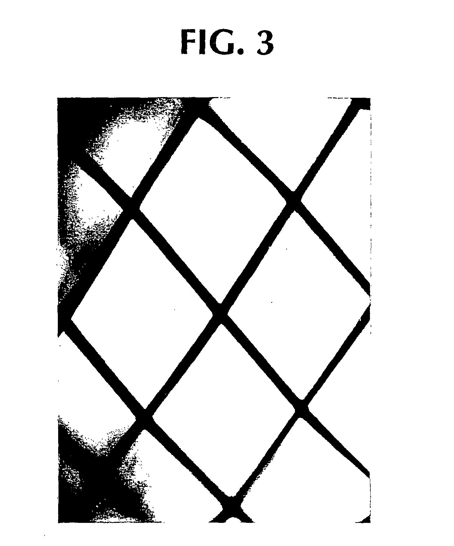

FIGURE 3 is a light microscopic photograph of a typical uncoated stent structure configuration (20X);
FIGURE 4A is a scanning electron microscope photograph (SEM) of a heparin containing poly siloxane coating on a stent in accordance with the invention (X20) after release of heparin into buffer for 49 days;
FIGURE 4B is a higher powered scanning electron microscopic photograph (SEM) of the coating of Figure 4A (X600);
FIGURE 5A is another scanning electron microscopic photograph (SEM) of a different stent coated with coating as produced with heparin incorporated into the polysiloxane (X20);
FIGURE 5B is an enlarged scanning electron microscopic photograph (SEM) of the coating of Figure 5B (X600);
FIGURE 6A is a light microscopic picture (X17.5) of a histologic cross-section of a silicone/heparin coated stent implanted in a swine coronary for 1 day;
FIGURE 6B depicts a pair of coated filaments of the stent of Figure 6A (X140) showing the open porous structure of the silicone;
FIGURE 7A is a scanning electron microscope photograph (SEM) that depicts a polysiloxane coating containing 5% dexamethasone (X600);
FIGURE 7B depicts the coating of Figure 7A (SEM X600) after dexamethasone release in polyethylene glycol (PEG 400/H₂O) for three months;
FIGURE 8 is a plot showing the total percent heparin released over 90 days from a coated stent in which the coated layer is 50% heparin (based on the total weight of the coating) in a silicone polymer matrix; release took place in phosphoric buffer (pH=7.4) at 37°C; and
FIGURE 9 is a plot of the total percent dexamethasone released over 100 days for two percentages of dexamethasone in silicon coated stents; release took place in polyethylene glycol (PEG), MW=400 (PEG 400/H₂O, 40/60, vol/vol) at 37°C.

### DETAILED DESCRIPTION

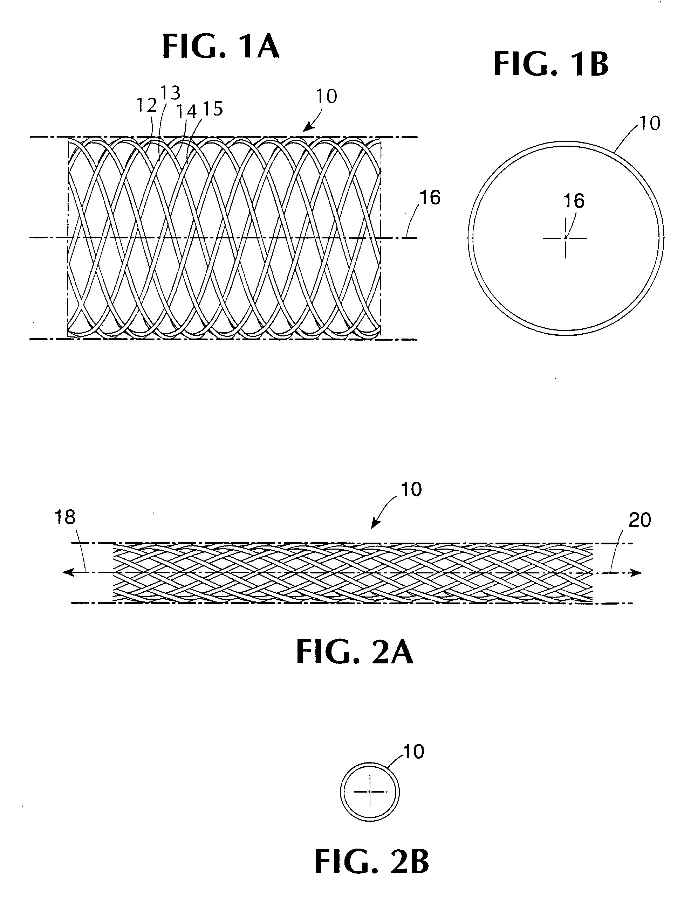

A type of stent device of one class designed to be utilized in combination with coatings in the present invention is shown diagrammatically in a side view and an end view, respectively contained in Figures 1A and 1B. Figure 1A shows a broken section of a generally cylindrical tubular body 10 having a mantle surface formed by a number of individual thread elements 12, 14 and 13, 15, etc. of these elements, elements 12, 14, etc. extend generally in an helix configuration axially displaced in relation to each other but having center line 16 of the body 10 as a common axis. The other elements 13, 15, likewise axially displaced, extend in helix configuration in the opposite direction, the elements extending in the two directions crossing each other in the manner indicated in Figure 1A. A tubular member so concerned and so constructed can be designed to be any convenient diameter, it being remembered that the larger the desired diameter, the larger the number of filaments of a given wire diameter (gauge) having common composition and prior treatment required to produce a given radial compliance.

The braided structure further characteristically readily elongates upon application of tension to the ends axially displacing them relative to each other along center line 16 and correspondingly reducing the diameter of the device. This is illustrated in Figures 2A and 2B in which a segment of the device 10 of Figures 1A and 1B has been elongated by moving the ends 18 and 20 away from each other in the direction of the arrows. Upon the release of the tension on the ends, the structure 10, if otherwise unrestricted, will reassume the relaxed or unloaded configuration of Figures 1A and 1B.

The elongation/resumption characteristic flexibility of the stent device enables it to be slipped or threaded over a carrying device while elongated for transportation through the vascular or other relevant internal luminal system of a patient to the site of interest where it can be axially compressed and thereby released from the carrying mechanism, often a vascular catheter device. At the site of interest, it assumes an expanded condition held in place by mechanical/frictional pressure between the stent and the lumen wall against which it expands.

The elongation, loading, transport and deployment of such stents is well known and need not be further detailed here. It is important, however, to note that when one contemplates coatings for such a stent in the manner of the present invention, an important consideration resides in the need to utilize a coating material having elastic properties compatible with the elastic deforming properties residing in the stent that it coats. The material of the stent should be rigid and elastic but not plastically deformable as used. As stated above, the preferred materials for fabricating the metallic braided stent include stainless steel, tantalum, titanium alloys including nitinol and certain cobalt-chromium alloys. The diameter of the filaments may vary but for vascular devices, up to about 10 mm in diameter is preferable with the range 0.01 to 0.05 mm.

Drug release surface coatings on stents in accordance with the present invention can release drugs over a period of time from days to months and can be used, for example, to inhibit thrombus formation, inhibit smooth muscle cell migration and proliferation, inhibit hyperplasia and restenosis, and encourage the formation of health neointimal tissue including endothelial cell regeneration. As such, they can be used for chronic patency after an angioplasty or stent placement. It is further anticipated that the need for a second angioplasty procedure may be obviated in a significant percentage of patients in which a repeat procedure would otherwise be necessary. A major obstacle to the success of the implant of such stents, of course, has been the occurrence of thrombosis in certain arterial applications such as in coronary stenting. Of course, antiproliferative applications would include not only cardiovascular but any tubular vessel that stents are placed including urologic, pulmonary and gastro-intestinal.

Various combinations of polymer coating materials can be coordinated with the braided stent and the biologically active agent of interest to produce a combination which is compatible at the implant site of interest and controls the release of the biologically active species over a desired time period. Preferred coating polymers include silicones (poly siloxanes), polyurethanes, thermoplastic elastomers in general, ethylene vinyl acetate copolymers, polyolefin rubbers, EPDM rubbers, and combinations thereof.

Specific embodiments of the present invention include those designed to elute heparin to prevent thrombosis over a period of weeks or months or to allow the diffusion or transport of dexamethasone to inhibit fibromuscular proliferation over a like period of time. Of course, other therapeutic substances and combinations of substances are also contemplated. The invention may be implanted in a mammalian system, such as in a human body.

The heparin elution system is preferably fabricated by taking finely ground heparin crystal, preferably ground to an average particle size of less than 10 microns, and blending it into a liquid, uncured poly siloxane/solvent material in which the blend (poly siloxane plus heparin) contains from less than 10% to as high as 80% heparin by weight with respect to the total weight of the material and typically the layer is between 10% and 45% heparin.

This material is solvent diluted and utilized to coat a metallic braided stent, which may be braided cobalt chromium alloy wire, in a manner which applies a thin, uniform coating (typically between 20 and 200 microns in thickness)of the heparin/polymer mixture on the surfaces of the stent. The polymer is then heat cured, or cured using low temperature thermal initiators (<100°C) in a room temperature vulcanization (RTV) process in situ on the stent evaporating solvent, typically tetrahydrofuran (THF) with the heparin forming interparticle paths in the silicone sufficiently interconnected to allow slow but substantially complete subsequent elution. The ultrafine particle size utilized allows the average pore size to be very small such that elution may take place over weeks or even months.

A coating containing dexamethasone is produced in a somewhat different manner. A poly siloxane material is also the preferred polymeric material. Nominally an amount equal to 0.4% to about 45% of the total weight of the layer of dexamethasone is used.

The dexamethasone drug is dissolved in a solvent, e.g., THF first. The solution is then blended into liquid uncured poly siloxane/solvent (xylene, THF, etc.) vehicle precursor material. Since the dexamethasone is also soluble in the solvent for the polysiloxane, it dissolves into the mixture. The coating is then applied to the stent and upon application, curing and drying, including evaporation of the solvent, the dexamethasone remains dispersed in the coating layer. It is believed that the coating is somewhat in the nature of a solid solution of recrystallized particles of dexamethasone in silicone rubber. Dexamethasone, as a rather small molecule, however, does not need gross pores to elute and may be transported or diffused outward through the silicone material over time to deliver its anti-inflammatory medicinal effects.

The coatings can be applied by dip coating or spray coating or even, in some cases, by the melting of a powdered form in situ or any other technique to which the particular polymer/biologically active agent combination is well suited.

It will be understood that a particularly important aspect of the present invention resides in the technology directed to the incorporation of very fine microparticles or colloidal suspensions of the drug into the polymer matrix. In the case of a crystalline drug, such as heparin, the drug release is controlled by the network the drug forms in the polymer matrix, the average particulate size controlling the porosity and so the ultimate elution rate.

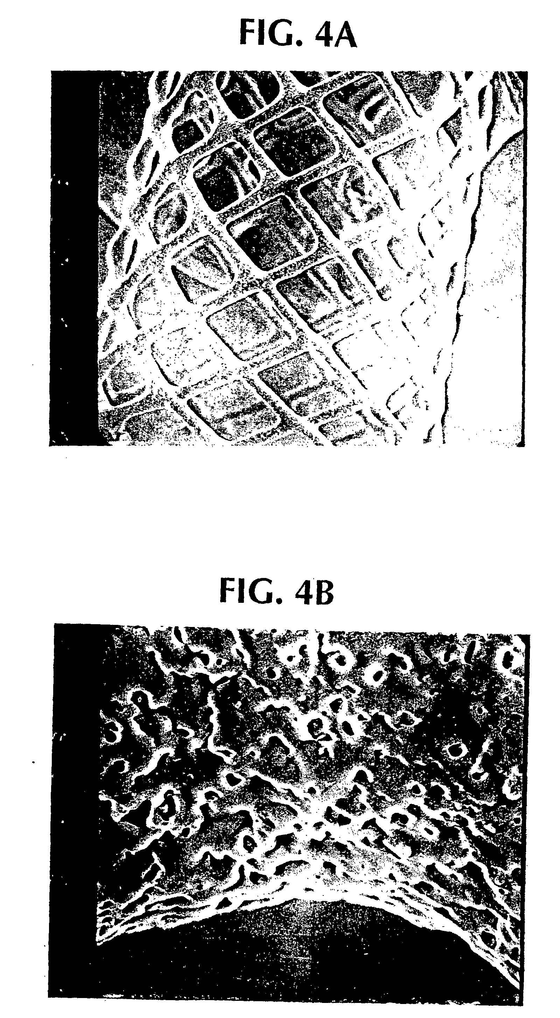

Figure 4A depicts a stent which has been spray coated with a solvent containing a cured polysilicone material including an amount of heparin crystals to provide a thin, uniform coating on all surfaces of the stent. The coated stent was cured at 150°C for 18 minutes; The sample was eluted in PBS for 49 days at 37°C and the stent was rinsed in ethanol prior to taking the scanning electron microscope picture of Figure 4A. Figure 4B shows a greatly enlarged (600X) scanning electron microscope photograph (SEM) of a portion of the coating of Figure 4A in which the microporosity is evident. The coating thickness may vary but is typically from about 75 to about 200 microns.

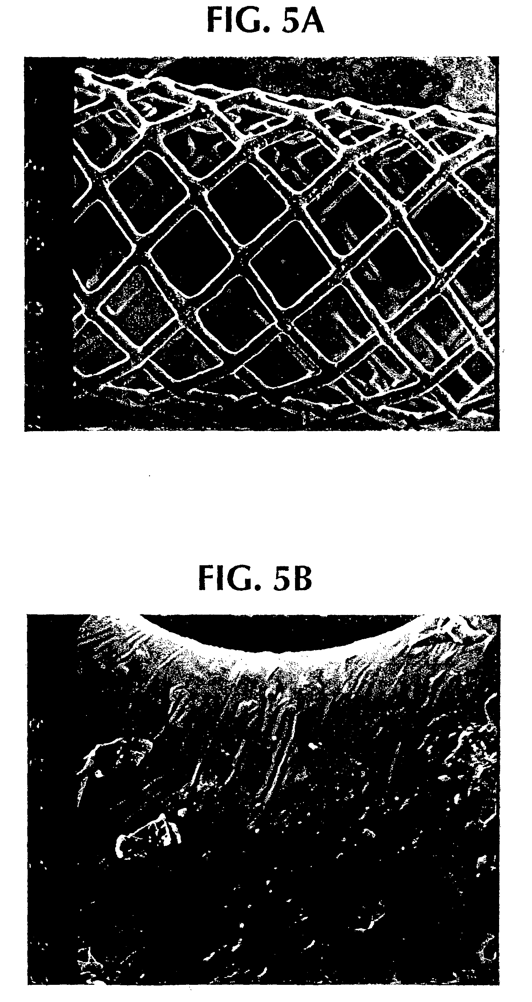

Figures 5A and 5B show scanning electron microscope photographs of a heparin containing polysiloxane stent. The Figure shows the coating prior to elution of the heparin. The coating was cured at 150° for 18 minutes. Figure 5B is greatly enlarged photograph (SEM) of a fragment of the coated surface of Figure 5A showing the substantially non-porous surface prior to elution.

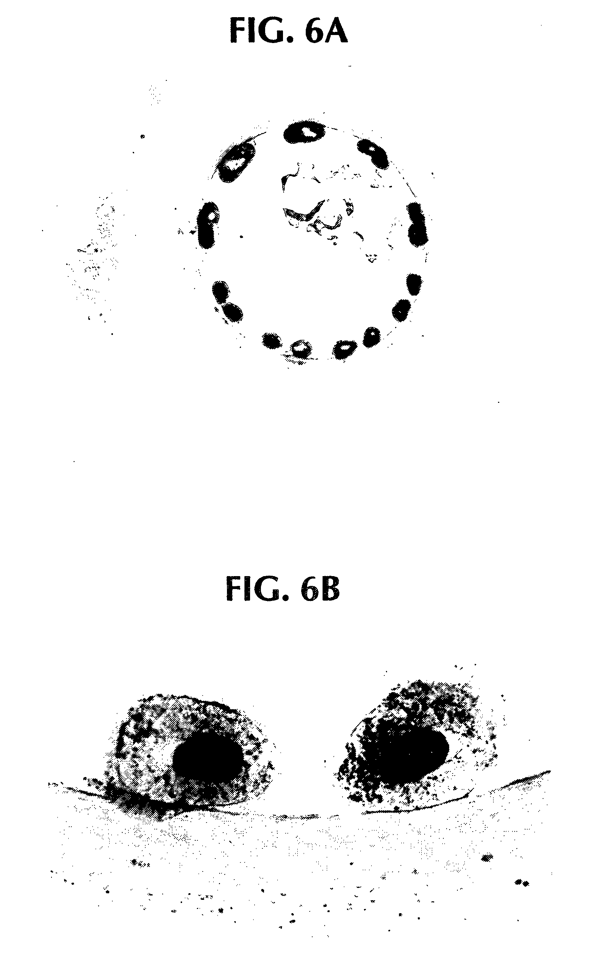

Figures 6A and 6B show the posture of a stent in accordance with the invention as implanted in a swine coronary. The blemish shown in Figure 6A represents a histological artifact of unknown origin. As can be seen in Figure 6B, the general texture of the heparin-containing silicone material appears as a relatively open matrix containing a large number of gross pores.

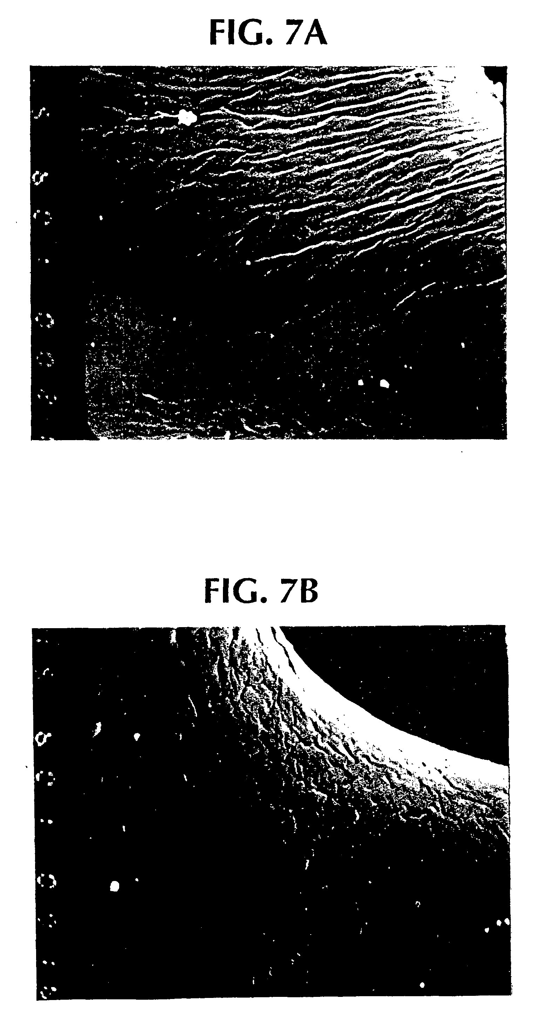

The substantially non-porous surface of Figure 7A typically occurs with an incorporation of an amount of non-particulate material such as dexamethasone which partially or entirely dissolves in the solvent for the poly siloxane prior to coating and cure. Upon curing of the polymer and evaporation of the solvent, depending on the loading of dexamethasone, the dexamethasone reprecipitates in a hydrophobic crystalline form containing dendrite or even elongated hexagonal crystals approximately 5 microns in size.

As can be seen in Figure 7B, even after release of the incorporated material or three months, the coating surface remains substantially non-porous indicating the transport or diffusion of the drug outward through the silicone material neither requires nor produces gross pores. The dexamethasone is incorporated in its more hydrophobic form rather than in one of the relatively more hydrophilic salt forms such as in a phosphate salt, for example.

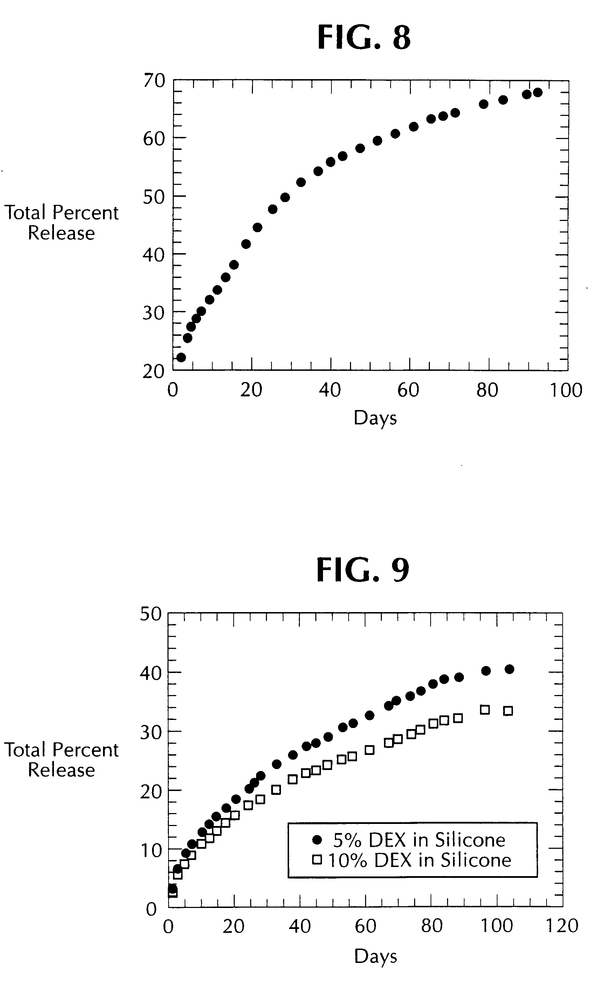

Figures 8 and 9 depict plots of total percent drug release related to long-term drug release stent coating layers. Figure 8 depicts the release of heparin from a 50% heparin loading in silicone. The silicone was cured at 90°C for 16 hours. The heparin release took place in a phosphoric buffer (pH=7.4) for 90 days at 37°C. The heparin concentration in the phosphoric buffer was analyzed by Azure A assay.

Figure 9 depicts a graphical analysis, similar to that depicted for heparin in Figure 8, for the release of dexamethasone at two different concentrations, i.e., 5% and 10% in silicone polymer. The coated stents were cured at 150°C for 20 minutes and the release took place in a polyethylene glycol (PEG), MW=400/water solution at 37°C ((PEG 400/H₂O) (40/60, vol/vol)). The dexamethasone concentrations were analyzed photometrically at 241 µm.

Figures 8 and 9 illustrate possible stent layer polymer/bioactive species combinations for long-term release. As stated above, the release rate profile can be altered by varying the amount of active material, the coating thickness, the radial distribution of bioactive materials, the mixing method, and the crosslink density of the polymer matrix. Sufficient variation is possible such that almost any reasonable desired profile can be simulated.

As stated above, while the allowable loading of the elastomeric material with heparin may vary in the case of silicone materials, heparin may exceed 60% of the total weight of the layer. However, the loading generally most advantageously used is in the range from about 10% to 45% of the total weight of the layer. In the case of dexamethasone, the loading may be as high as 50% or more of the total weight of the layer but is preferably in the range of about 0.4% to 45%.

It will be appreciated that the mechanism of incorporation of the biologically active species into a thin surface coating structure applicable to a metal stent is an important aspect of the present invention. The need for relatively thick-walled polymer elution stents or any membrane overlayers associated with many prior drug elution devices is obviated, as is the need for utilizing biodegradable or reabsorbable vehicles for carrying the biologically active species. The technique clearly enables long-term delivery and minimizes interference with the independent mechanical or therapeutic benefits of the stent itself.

Coating materials are designed with a particular coating technique, coating/drug combination and drug infusion mechanism in mind. Consideration of the particular form and mechanism of release of the biologically active species in the coating allow the technique to produce superior results. In this manner, delivery of the biologically active species from the coating structure can be tailored to accommodate a variety of applications.

Whereas the polymer of the coating may be any compatible biostable elastomeric material capable of being adhered to the stent material as a thin layer, hydrophobic materials are preferred because it has been found that the release of the biologically active species can generally be more predictably controlled with such materials. Preferred materials include silicone rubber elastomers and biostable polyurethanes specifically.

This invention has been described herein in considerable detail in order to comply with the Patent Statutes and to provide those skilled in the art with the information needed to apply the novel principles and to construct and use embodiments of the example as required. However, it is to be understood that the invention can be carried out by specifically different devices and that various modifications can be accomplished without departing from the scope of the invention itself.

## Claims

1. A stent for implantation in a body comprising a tubular metal body (10) having open ends and a sidewall and a layer on a surface of this sidewall, said layer comprising a matrix material incorporating an amount of biologically active material therein for timed delivery therefrom **characterised in that** the sidewall is an open lattice structure with openings therein and the matrix material comprises a hydrophobic elastomeric material which permits the layer to adheringly conform to the lattice sidewall structure and so leave the openings substantially free of layer material and whereby said layer is from about 75µm to about 200µm in thickness.

2. A stent according to claim 1 **characterised in that** the tubular body (10) is formed of an open braid of filaments (12, 13, 14, 15) of fine metallic wire which is axially deformable for insertion but which resumes a predetermined diameter and length upon relaxation.

3. A stent according to claim 1 or 2 **characterised in that** the metal is selected from the group consisting of stainless steel, titanium alloys including nitinol, tantalum and cobalt-chrome alloys.

4. A stent according to any one of claims 1 to 3 **characterised in that** the layer is applied as a solvent mixture of uncured polymeric material and finely divided biologically active species and then cured at an elevated temperature.

5. A stent according to any one of claims 1 to 4 **characterised in that** the elastomeric material is selected from the group consisting of silicones, polyurethanes, thermoplastic elastomers, ethylene vinyl acetate copolymers, polyolefin elastomers, EPDM rubbers and combinations thereof.

6. A stent according to any one of claims 1 to 5 **characterised in that** the elastomeric material is a poly siloxane and the biologically active material is selected from the group consisting of heparin and dexamethasone.

7. A stent according to claim 6 wherein the biologically active material is heparin **characterised in that** the amount of heparin is from about 10% to about 45% of the total weight of the layer.

8. A stent according to claim 4 **characterised in that** the biologically active species is at least partially soluble in the solvent mixture of uncured polymeric material.

9. A stent according to claim 8 **characterised in that** the biologically active species is dexamethasone and comprises from about 0.4% to about 45% of the total weight of the layer.

10. A stent according to any one of claims 1 to 9 **characterised in that** the layer is adapted to provide long-term delivery of the biologically active material in the body.

11. A stent according to any one of claims 1 to 10 **characterised in that** the biologically active material is an agent inhibiting hyperplasia.

## Patentansprüche

1. Dilatator zur Implantation in einen Körper, umfassend einen röhrenförmigen Metallkörper (10) mit offenen Enden und einer Seitenwand und einer Schicht auf einer Oberfläche dieser Seitenwand, wobei die Schicht ein Matrixmaterial umfasst, das eine Menge an biologisch wirksamem Material darin zur zeitbestimmten Abgabe daraus enthält, **dadurch gekennzeichnet, dass** die Seitenwand eine offene Gitterstruktur darstellt, mit Öffnungen darin und das Matrixmaterial ein hydrophobes, elastomeres Material umfasst, das gestattet, dass die Schicht sich haftend an die Gitterseitenwandstruktur anschmiegt und so die Öffnungen im Wesentlichen frei von Schichtmaterial belässt und wobei die Schicht etwa 75 µm bis etwa 200 µm in der Dicke ist.

2. Dilatator nach Anspruch 1, **dadurch gekennzeichnet, dass** der röhrenförmige Körper (10) aus einem offenen Geflecht von Filamenten (12, 13, 14, 15) aus feinem Metalldraht gebildet ist, der axial zum Einsatz verformbar ist, der allerdings einen vorbestimmten Durchmesser und eine vorbestimmte Länge nach Entspannung wiedererlangt.

3. Dilatator nach Anspruch 1 oder 2, **dadurch gekennzeichnet, dass** das Metall aus der Gruppe, bestehend aus Edelstahl, Titanlegierungen, einschließlich Nitinol, Tantal und Kobaltchromlegierungen, ausgewählt ist.

4. Dilatator nach einem der Ansprüche 1 bis 3, **dadurch gekennzeichnet, dass** die Schicht als ein Lösungsmittelgemisch von ungehärtetem Polymermaterial und fein verteilten, biologisch wirksamen Spezies aufgetragen wird und dann bei erhöhter Temperatur gehärtet wird.

5. Dilatator nach einem der Ansprüche 1 bis 4, **dadurch gekennzeichnet, dass** das elastomere Material aus der Gruppe, bestehend aus Siliconen, Polyurethanen, thermoplastischen Elastomeren, Ethylen-Vinylacetat-Copolymeren, Polyolefinelastomeren, EPDM-Kautschuken und Kombinationen davon, ausgewählt ist.

6. Dilatator nach einem der Ansprüche 1 bis 5, **dadurch gekennzeichnet, dass** das elastomere Material ein Polysiloxan ist und das biologisch wirksame Material aus der Gruppe, bestehend aus Heparin und Dexamethason, ausgewählt ist.

7. Dilatator nach Anspruch 6, wobei das biologisch wirksame Material Heparin ist, **dadurch gekennzeichnet, dass** die Menge an Heparin von etwa 10 % bis etwa 45 % des Gesamtgewichts der Schicht beträgt.

8. Dilatator nach Anspruch 4, **dadurch gekennzeichnet, dass** die biologisch wirksame Spezies mindestens teilweise in dem Lösungsmittelgemisch von ungehärtetem Polymermaterial löslich ist.

9. Dilatator nach Anspruch 8, **dadurch gekennzeichnet, dass** die biologisch wirksame Spezies Dexamethason ist und etwa 0,4 % bis etwa 45 % des Gesamtgewichts der Schicht umfasst.

10. Dilatator nach einem der Ansprüche 1 bis 9, **dadurch gekennzeichnet, dass** die Schicht zur Bereitstellung einer Langzeitabgabe des biologisch wirksamen Materials in dem Körper angepasst ist.

11. Dilatator nach einem der Ansprüche 1 bis 10, **dadurch gekennzeichnet, dass** das biologisch wirksame Material ein Hyperplasie inhibierendes Mittel ist.

## Revendications

1. Extenseur pour l'implantation dans un corps comprenant un corps métallique tubulaire (10) ayant des extrémité ouverte et une paroi latérale et une couche sur une surface de cette paroi latérale, ladite couche comprenant un matériau de matrice incorporant une quantité de matériau biologiquement actif dans celle-ci, pour une administration dans le temps depuis celle-ci, **caractérisée en ce que** la paroi latérale est une structure en treillage ouvert avec des ouvertures dans celle-ci, et le matériau de matrice comprend un matériau d'élastomère hydrophobe qui permet à la couche de se conformer par adhérence à la structure de la paroi latérale du treillage et de laisser aussi des ouvertures substantiellement libres du matériau de couche, et de ce fait, ladite couche a une épaisseur d'environ 75 µm à environ 200 µm.

2. Extenseur selon la revendication 1, **caractérisé en ce que** le corps tubulaire (10) est formé d'une tresse ouverte de filaments (12, 13, 14, 15) de fil métallique fin qui est déformable de façon axiale pour l'insertion, mais qui reprend un diamètre et une longueur prédéterminés lors du relâchement.

3. Extenseur selon les revendications 1 ou 2, **caractérisé en ce que** le métal est choisi dans le groupe constitué par l'acier inoxydable, les alliages de titane y compris le nitinol, le tantale et les alliages cobalt-chrome.

4. Extenseur selon l'une quelconque des revendications 1 à 3, **caractérisé en ce que** la couche est appliquée sous forme d'un mélange de solvant de matériaux polymériques non-vulcanisés et d'espèce biologiquement active finement divisée, et ensuite vulcanisé à une température élevée.

5. Extenseur selon l'une quelconque des revendications 1 à 4, **caractérisé en ce que** le matériau d'élastomère est choisi dans le groupe constitué par les silicones, les polyuréthanes, les élastomères thermoplastiques, les copolymères d'acétate d'éthylène de vinyle, les élastomères de polyoléfine, les caoutchoucs de EPDM et des combinaison de ceux-ci.

6. Extenseur selon l'une quelconque des revendications 1 à 5, **caractérisé en ce que** le matériau d'élastomère est un polysiloxane et le matériau biologiquement actif est choisi dans le groupe constitué par l'héparine et la dexaméthasone.

7. Extenseur selon la revendication 6, dans lequel le matériau biologiquement actif est l'héparine, **caractérisée en ce que** la quantité d'héparine est d'environ 10 % à environ 45 % du poids total de la couche.

8. Extenseur selon la revendication 4, **caractérisé en ce que** l'espèce biologiquement active est au moins partiellement soluble dans le mélange de solvant des matériaux polymériques non-vulcanisés.

9. Extenseur selon la revendication 8, **caractérisé en ce que** l'espèce biologiquement active est la dexaméthasone et comprend environ 0,4 % à environ 45 % du poids total de la couche.

10. Extenseur selon l'une quelconque des revendications 1 à 9, **caractérisé en ce que** la couche est adaptée pour fournir une administration à long terme du matériau biologiquement actif dans le corps.

11. Extenseur selon l'une quelconque des revendications 1 à 10, **caractérisé en ce que** le matériau biologiquement actif est un agent inhibant l'hyperplasie.
